# EUROPEAN PATENT SPECIFICATION

(11) **EP 2 043 513 B1**
(45) Date of publication and mention of the grant of the patent: **26.03.2014**
(21) Application number: 07813182.8
(22) Date of filing: 20.07.2007
(51) Int. Cl.: A61B 5/0215, A61B 5/07, A61F 2/82

(54) **MULTIPLE SENSOR DEPLOYMENT**
EINSATZ MEHRERER SENSOREN
DÉPLOIEMENT DE MULTIPLES CAPTEURS

(30) Priority: 21.07.2006 US 820050 P; 21.07.2006 US 820059 P
(43) Date of publication of application: 08.04.2009
(73) Proprietor: Cardiac Pacemakers, Inc., St. Paul MN 55112 (US)
(72) Inventor: HUELSKAMP, Paul J., St. Paul, Minnesota 55108 (US)
(74) Representative: Butenschön, Antje
(86) International application number: PCT/US2007/074020
(87) International publication number: WO 2008/011592

(56) References cited:
- US-A- 6 141 588
- US-A1- 2002 183 628
- US-A1- 2003 158 584
- US-A1- 2004 117 204
- US-A1- 2004 199 238
- US-A1- 2005 080 346
- US-A1- 2006 031 378
- US-B1- 6 277 078

## Description

### TECHNICAL FIELD

The present invention relates to sensors that are implantable within the human body. More specifically, the invention relates to devices and methods for implanting multiple sensors within the human body.

### BACKGROUND

US Patent US 6,277,078 B1 discloses an intra-body implantable system for monitoring parameters associated with heart performance. The system includes a first sensor being implantable within the heart for collecting information pertaining to a pressure in a first cavity of the heart, an additional sensor being implantable in a blood vessel supporting blood flow into or out of a second cavity of the heart (the second sensor also being for collecting information pertaining to a pressure) and at least one device implantable in the body and being in data communication with the two sensors. The latter device then relays the information pertaining to the pressure outside the body.

Also known (US 2002/0183628 A1) is an endovascular implant or endograft including a tubular sleeve having integral inner and outer layers. A pressure sensor is embedded between the two layers and is covered thereby. The sleeve is flexible at the pressure sensor to permit transfer of pressure through the sleeve for detection by the pressure sensor in use.

US 2006/0031378 A1 describes a system for providing digital data communications over a wireless intra-body network. Identifiers are uniquely assigned to a plurality of implantable devices. A slave implantable device is activated in response to an activation signal transmitted through the wireless interface by a master implantable device. A wireless communications link is established between the slave implantable device and the master implantable device upon matching of the identifier assigned to the slave implantable device.

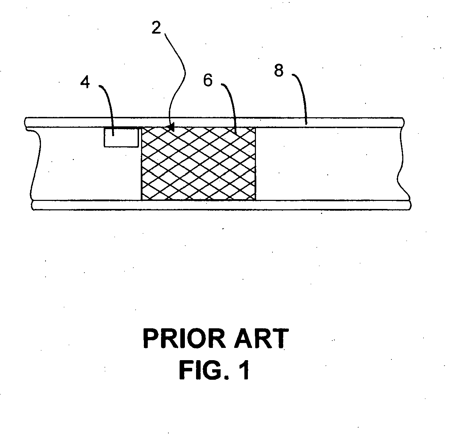

US 6,141,588 describes a medical system comprising a control device ("planet") and a plurality of sensing and stimulating devices ("satellites"). The planet individually commands each satellite to deliver pacing energy to the heart. The satellites are capable of determining when a sense event has occurred at the side of said satellite and transmitting an encoded signal to the planet indicating that a sense event has occurred along with an identifying code indicating to the planet which satellite detected the sense event. Medical sensors can be implanted into the human body to retrieve diagnostic data regarding certain bodily functions. For example, pressure sensors located within the vascular system of the body can measure blood pressure at various locations within the body. FIG. 1 illustrates a representative sensor assembly 2 having a sensor 4 and a compressible retention member 6 positioned within a blood vessel 8. The sensor 4 is a self-contained device, having its own power supply and communication circuitry for communicating data to a remote device. Sensors of this type can be implanted into the body by inserting a catheter into the body and moving the catheter through the cardio-vascular system until the end of the catheter is positioned at the desired implant location. The sensor can then be fed through the catheter and positioned within the appropriate blood vessel.

Due to the nature of the human body, portions of the vascular system through which the catheter is navigated can have relatively acute radii, and thus the catheter may be required to make one or more turns within the body before reaching the desired implant location. Thus, any sensor that is fed through the body must be small enough to negotiate any turns in the catheter when it is positioned within the body. This size limitation impacts the size of the power supply contained within the sensor and correspondingly, impacts the life of the power supply and thus the amount of time that a sensor can function within the body. For example, a sensor may have a power supply with a rated life of 10 years under normal use, including periodic recharging. It is not desirable to replace sensors of this type within the body. Therefore, it is desirable to be able to extend the length of time that diagnostic data can be retrieved at a given site without requiring replacement of sensors. What is needed, then, is a way to measure diagnostic data at a given site for longer periods of time, given the constraints of size on diagnostic sensors.

### SUMMARY

In order to understand the present invention, a method of measuring pressure within the human body is described. The method includes implanting a pressure sensing assembly into a human body. The sensing assembly includes a flexible structure capable of expanding when implanted and first and second sensor members each having self contained power supplies. The method includes performing periodic data collection events, including collecting a sensor data packet from at least one of the first and second members.

The invention is directed toward a sensor assembly for implantation into a human body. The sensor assembly includes a flexible structure capable of expanding when implanted within the human body and first and second sensor members. The first sensor member includes a self-contained power supply, a sensing element and an integral communication device capable of communicating with a remote communication device. The sensor assembly is, in one aspect, part of a pressure sensing system implanted within a human body. The pressure sensing system includes a remote communication device implanted within the human body. In one aspect of the invention, the remote communication device is a pulse generator. The remote communication device is capable of broadcasting messages to the sensors. Broadcast messages include address data that is recognized by the intended sensor members. In order to do so, the remote communication device is operable to transmit a sensor data request message to the first and second pressure sensor members, the sensor data request message configured to prompt each pressure sensor member to transition from a power save state to an initialization state for processing incoming communication data; and each pressure sensor member is operable to compare an address in the sensor data request message against a unique address and, upon receiving a matching address, transition from the initialization state to an active state in which said sensor member is activated for sensing pressure data.

Additionally, the remote communication device is operable to collect and process a sensor data packet transmitted from one of the sensor members, the sensor data packet including a power supply value describing the charge level of a power supply of the one of the sensor members.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

### BRIEF DESCRIPTION OF THE DRAWINGS

FIG. 1 is a cross-sectional fragmentary view of a cavity within the human body with a prior art implantable sensor assembly positioned therein.
FIG. 2 illustrates an implantable multi-sensor assembly that can be used in relation to embodiments of the present invention.
FIG. 3 is a functional block diagram of a sensor element of the multi-sensor assembly of FIG. 2.
FIG. 4 is a cross-sectional view of the implantable multi-sensor assembly of FIG. 2 positioned at a radius within an insertion tool.
FIG. 5 is a flow diagram of a method of communication between the sensors of the multi-sensor assembly and a remote communication device according to one embodiment of the invention.
FIG. 6 is a flow diagram of a method of communication between the sensors of the multi-sensor assembly and a remote communication device according to another embodiment of the invention.
FIG. 7 is a flow diagram of a method of communication between the sensors of the multi-sensor assembly and a remote communication device according to yet another embodiment of the invention.

While the invention is amenable to various modifications and alternative forms, specific embodiments have been shown by way of example in the drawings and are described in detail below. The intention, however, is not to limit the invention to the particular embodiments described. On the contrary, the invention is intended to cover all modifications, equivalents, and alternatives falling within the scope of the invention as defined by the appended claims.

### DETAILED DESCRIPTION

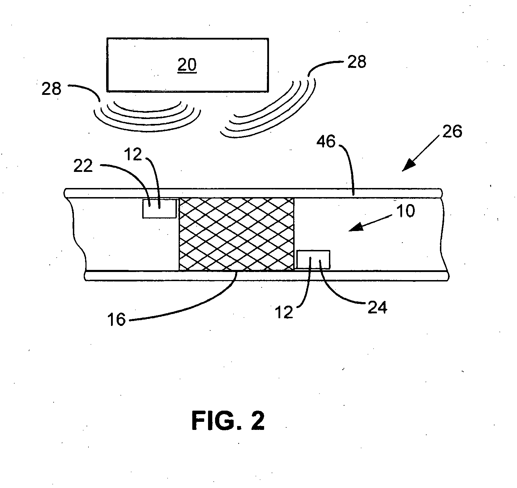

FIG. 2 illustrates a sensor assembly 10 having a plurality of sensor members 12 each coupled to a compressible retention device 16 implanted into a blood vessel 26 of a human body according to one embodiment of the invention. The sensor assembly 10, as described below, includes two or more sensor members 12 and is capable of being implanted within the cardiovascular system of the human body to measure blood pressure at the implant location. The sensor assembly 10 is shown positioned within a blood vessel 26 within a human body. The compressible retention device 16 is expanded to contact the inner surface 46 of the blood vessel 26 to retain the sensor assembly 10 in its proper position. The compressible retention device 16 can be a stent, a strut, or other similar devices. Each of the sensor members 12 are coupled to the compressible retention device 16 by welding the sensor members 12 to the retention device 16 or by using other known coupling structures. The sensor assembly 10 is shown having two sensor members 12, although the sensor assembly can have any number of additional sensor members without departing from the scope of the invention. For example, the sensor assembly 10 could have a third sensor member (not shown) coupled to the compressible retention device 16.

When the sensor assembly 10 is implanted within the human body as shown in FIG. 2, the sensors 12 are capable of communicating with a remote communication device 20 via the communication link 28. The remote communication device 20, in one embodiment, is a pulse generator implanted within the human body. Alternatively, the remote communication device 20 can be any other device, either implanted within or externally located from the human body. In one embodiment, the communication link 28 between the sensors 12 and the communication device 20 is established using wireless acoustic communication. Alternatively, the communication link 28 can be established through inductive, radio frequency, or other communication technologies.

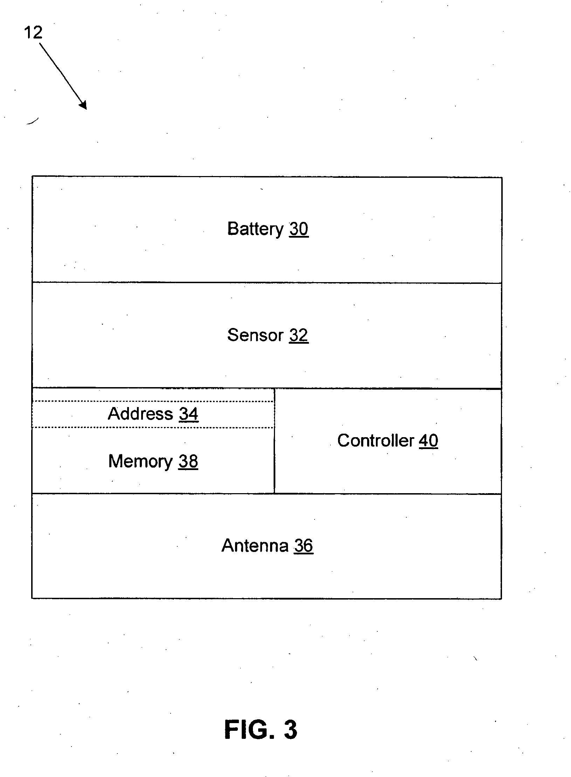

Sensors 12 of the sensor assembly 10 are further identified as a first sensor member 22 and a second sensor member 24. For the purposes of this specification, the sensor members 12 of the sensor assembly 10 are referred to collectively or generically as sensor members 12, but individually and specifically as a first sensor member 22 and a second sensor member 24. FIG. 3 is a block diagram of the sensor member 12. The sensor member 12 includes a rechargeable power supply 30, a sensing element 32, a memory 38, including address data 34, a communication element 36, and a controller 40. The communication element 36 includes appropriate structure to communicate with the remote communication device 20 (FIG. 2) and can include one or more communication technologies of the types listed above and/or others. The power supply 30 can be a battery.

The memory 38 can be any type of memory known in the art. By way of example, but not limitation, the memory 38 can include random access memory (RAM) or read only memory (ROM). The memory 38 could include electrically erasable programmable ROM (EEPROM). Other types of memory may be known in the art that may be suitable for particular implementations. The controller 40 could be, without limitation, a microcontroller, a microprocessor, a digital signal processor, or an application specific integrated circuit (ASIC). The controller 40 may have registers for temporary storage of data or executable instructions, which may be read from memory 38.

The address data 34 provides identification within the sensor member 12 to allow the sensor member 12 to determine whether the sensor member 12 is the intended recipient of a messages or data. Messages from the communication device 20 may be broadcast messages or targeted to particular sensor members. Targeted messages sent from the remote communication device 20 include an address associated with the intended sensor member 12. Each sensor member 12 processes messages that have an address that corresponds to its unique address. Thus, the remote communication device 20 is capable of directing a message to one or all of the sensor members 12. In one embodiment, the address data 34 is stored in reprogrammable or read only digital memory. It is anticipated that the first sensor member 22 and the second sensor member 24 can have the same or similar construction. However, the first sensor member 22 and the second sensor member 24 can include some differences and other features without departing from the scope of the invention.

The sensor element 32 detects a physiologic parameter, such as, but not limited to, pressure, and emits a signal corresponding to the detected parameter. In this respect, the sensor element 32 may be piezo or capacitive in nature, or any other appropriate technology for sensing a physiologic parameter of interest. Although embodiments described herein relate primarily to sensing of blood pressure, it will be understood by those of skill in the art that other types of physiologic parameters may be sensed, depending on the particular application. The sensor member 12 is typically housed in a housing composed of a bio-compatible material to facilitate implantation in the human body.

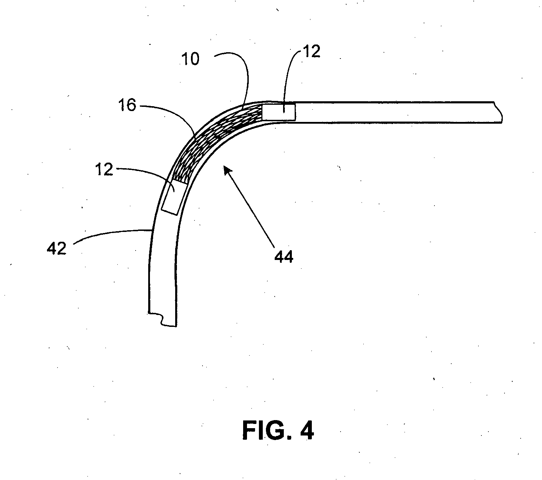

FIG. 4 illustrates a portion of an insertion tool 42 used to implant the sensor assembly 10 according to one embodiment of the invention. The insertion tool 42 can be a flexible catheter that is inserted into the vascular system of the human body and fed through to a desired implantation location within the human body. Once the insertion tool 42 is properly located within the human body, the sensor assembly 10 can be fed through the insertion tool 42 to the desired implantation location. When the sensor assembly 10 is loaded into the insertion tool 42, the compressible retention device 16 is compressed so that the sensor assembly 10 is capable of fitting within the insertion tool 42. As is shown in FIG. 4, the compressible retention member 16 is flexible enough to conform to a radius 44 and thus both of the sensor members 12 are able to pass though the radius. Once the sensor assembly 10 is fed through the insertion tool 42, it is located in the blood vessel 26 (FIG. 2). The compressible retention member 16 expands to engage the inner surface 46 of the blood vessel 26 to secure the sensor assembly 10 into position. Alternatively, the sensor assembly can be implanted in other portions of the human body, including, but not limited.to, the esophagus and the air canal.

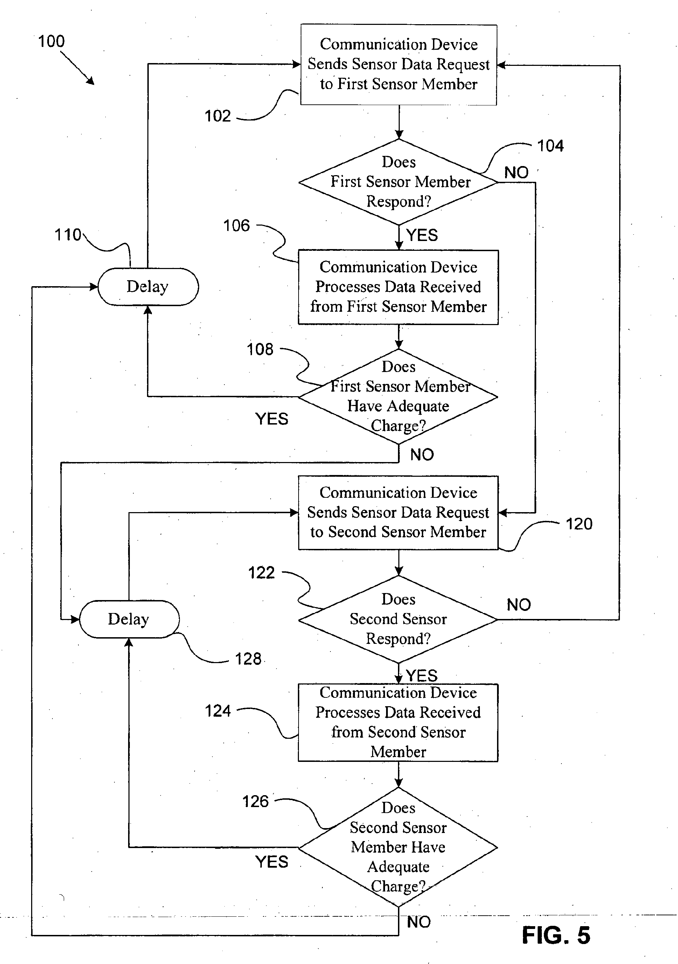

FIG. 5 is a functional flowchart 100 that illustrates a method of communicating sensor data packets between the remote communication device 20 (FIG. 2) and the sensor members 12 (FIG. 2) according to one embodiment of the invention. The remote communication device 20 and the sensor members 12 have a master/slave relationship such that the sensor members 12 rely on a communication from the remote communication device 20 before they perform any data collection and/or transmission. Therefore, it is the remote communication device 20 that determines what and when steps are taken in the communication method illustrated in flowchart 100. Prior to any request from the remote communication device 20, the sensor members 12 are in a power save state. It is to be understood that the description of the data collection method below describes the functional aspects of the method and that numerous implementations can be used to achieve the described functions. For example, communication protocols can be used to define the structure of messages sent between the remote communication device 20 and the sensor members 12. One particular implementation of the communication protocol between the remote communication device 20 and sensor members 12 is described in detail in related US patent application publication US 2008/0021972 A1, which was filed on the same day as this application and claims priority to U.S. Provisional Patent Application Serial No. 60/820,059.

Referring to block 102 of flowchart 100, the remote communication device 20 seeks to acquire a sensor data packet from the first sensor member 22. The remote communication device 20 initiates communication with the first sensor member 22 either at a predetermined time or in response to a request from an external communication device (not shown). The remote communication device 20 broadcasts a sensor data request message requesting sensor data from the first sensor member 22 (FIG. 2). The remote communication device 20 then waits a predetermined amount of time for the first sensor member 22 to respond. The data request message can request a single sensor data point, multiple sensor data points, or a continuous stream of data for a given period of time.

When a sensor data request message requesting sensor data from the first sensor member 22 is broadcast, all of the sensor members 12 receive the message and enter an identification state. When the sensor member(s) 12 is in an initialization state, the sensor member(s) 12 processes incoming communication data to determine whether the message is intended for that particular sensor. The sensor data request message from the remote communication device 20 includes an address. Each of the sensor members 12 compares the address in the sensor data request message to determine whether the address corresponds to that sensor member's address. If the sensor member(s) 12 determines that the addresses don't match, the sensor member(s) 12 ignores the message and returns to the power save state.

The sensor data request message of block 102 includes an address that corresponds to the unique address data 38 of the first sensor member 22. The first sensor member 22, recognizing that the address of the sensor data request message matches its unique address data 38 then enters into an active state in which the first sensor member 22 collects sensor data and transmits a sensor data packet back to the remote communication device 20. The sensor data packet, in one embodiment includes sensed data, such as pressure. In addition, the sensor data packet includes a power supply value that describes the charge level of its power supply. Once the first sensor member 22 has transmitted its data to the remote communication device 20, it returns to a power save state.

Referring to block 104, after sending the sensor data request message and waiting a predetermined length of time, if the remote communication device 20 has not received a sensor data packet from the first sensor member 22, the remote communication device 20 will request information from the second sensor member 24, as is illustrated in block 120 and described below. Before the remote communication device 20 sends a sensor data request message to the second sensor member 24, the remote communication device 20 may attempt more than once to collect information from the first sensor member 22. If, however, the remote communication device 20 does receive data from the first sensor member 22, the remote communication device 20 processes the sensor data packet received from the first sensor member 22 as illustrated in block 106 of flowchart 100.

The step of processing the sensor data packet can include storing the sensed data and/or power supply value, communicating the sensor data and/or power supply value to another remote device (not shown), using the sensor data and/or power supply value in calculations and/or algorithms, or any combination of the above. As shown in block 108, the remote communication device 20 next determines whether the first sensor member 22 has adequate charge left in its power supply 30 (Fig.3). The power supply value is compared against a charge threshold value to determine whether the first sensor member 22 has sufficient charge remaining to continue collecting sensor data and to transmit it to the remote communication device 20. If the power supply value is greater than the threshold value, the first sensor member 22 has adequate charge remaining and the remote communication device waits (represented as a delay in block 110) either for a predetermined amount of time or for a request from another remote device (not shown) to go to block 102 and repeat the data collection cycle. If, however, the power supply value is lower than the threshold value, the first sensor member 22 has an inadequate charge left in its power supply, and the remote communication device 20 waits (represented as a delay in block 128) for either a predetermined amount of time or for an external request to go to request data from the second sensor member 24.

Block 120 represents the request for sensor data from the second sensor member 24. The remote communication device 20 sends a sensor data request message having an address that corresponds to the unique address 38 of the second sensor member 24. The remote communication device 20 then waits for the second sensor member 24 to respond. If the second sensor member 24 receives the sensor data request message, it enters an active state, collects sensor data and the power supply value, transmits a data packet back to the remote communication device 20, and returns to a power save state.

Referring to block 122, after sending the sensor data request message and waiting a predetermined length of time, if the remote communication device 20 has not received a sensor data packet from the second sensor member 24, the remote communication device 20 will request information from the first sensor member 22, as is illustrated in block 102 and described above. Before the remote communication device 20 sends a sensor data request message to the first sensor member 22, the remote communication device 20 may attempt more than once to collect a sensor data packet from the second sensor member 24. It should be noted that the remote communication device 20 requests sensor data from the second sensor member 24 only after either not receiving a response to a data request from the first sensor member 22 or due to a low charge condition on the first sensor member 22. However, because the power supply is rechargeable, it is entirely possible that the first sensor member 22 has been recharged and will be able to respond to a request for sensor data despite being unable to do so previously. If, however, the remote communication device 20 receives a sensor data packet from the second sensor member 24, the remote communication device processes the data packet as illustrated in block 124. The sensed data received from the second sensor member 24 is processed in a similar manner to that described above with respect to the first sensor member 22.

As illustrated in block 126, the remote communication device 20 next determines whether the second sensor member 24 has adequate charge left in its power supply 30. The power supply value of the second sensor member 24 is compared against the charge threshold value. If the power supply value is greater than the threshold value, the second sensor member 24 has adequate charge left in its power supply 30 and the remote communication device 20 waits (represented as a delay in block 128) either a predetermined amount of time, or for an external request to prompt the remote communication device to request data from the second sensor member 24 (as illustrated in block 120).

If, however, the power supply value is lower than the threshold value, the second sensor member 24 has an inadequate charge left in its power supply and the remote communication device 20 waits (represented as a delay in block 110) for a prompt to request sensor data from the first sensor member 22 (as illustrated in block 102). Alternatively, the remote communication device 20 may from time to time jump from block 128 to block 102 by issuing a sensor data request message with an address corresponding to the unique address 38 of the first sensor member 22 in attempt to request data from the first sensor member 22 even though the second sensor member 24 is working properly and has sufficient charge (not shown in flowchart 100). While flowchart 100 details the interaction between the remote communication device 20 and a sensor assembly 10 having two sensor members 12, other embodiments can include any number of sensor members 12. In those instances, the remote communication device 20 can move from one sensor to the next to get readings from a sensor with adequate charge in a way similar to that described here. Further, while flowchart 100 and the description above detail the transmission of a single sensor data packet transmission per sensor data request, a single data request could alternatively request a plurality of sensor data packets to provide readings over a period of time.

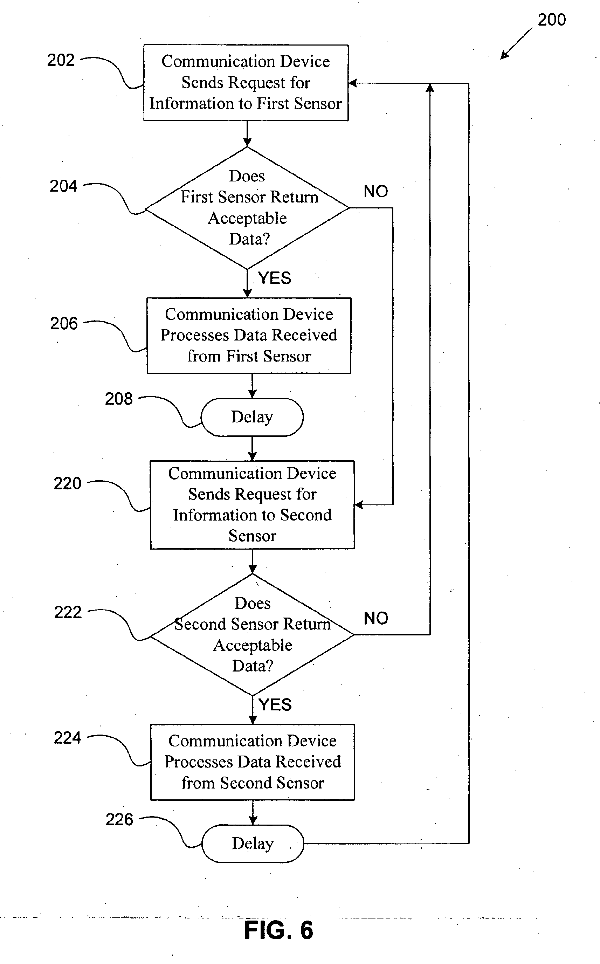

FIG. 6 is a functional flowchart 200 that illustrates a method of communication of sensor data between the remote communication device 20 and the sensor members 12 (shown in FIG. 2) according to another embodiment of the invention. In this embodiment, the remote communication device 20 alternates data collection between the first sensor member 22 and the second sensor member 24 (and any additional sensors) rather than collecting data from just one sensor until that sensor fails to respond or signals a low charge.

At block 202 of flowchart 200, the remote communication device 20 seeks to acquire sensor data from the first sensor member 22. The remote communication device 20 initiates communication with the first sensor member 22 by broadcasting a data request message having an address corresponding to the unique address 38 of the first sensor member 22 as is described above with respect to block 102 of flowchart 100. The first sensor member 22 responds as described above by providing a sensor data packet to the remote communication device 20 and then returns to a power save state.

Referring to block 204, after sending the sensor data request message and waiting a predetermined length of time, if the remote communication device 20 has not received a sensor data packet from the first sensor member 22, the remote communication device will request information from the second sensor member 24, as is illustrated in block 220 and described below. As in step 104 of flowchart 100, if the first sensor member 22 has not responded in a timely manner, the remote communication device 20 may attempt more than one request for information before attempting to communicate with the second sensor member 24.

If the remote communication device 20 receives timely data from the first sensor member 22, the remote communication device 20 processes the data at block 206. The step of processing the sensor data packet can include storing the sensed data and/or power supply value, communicating the sensed data and/or power supply value to another remote device (not shown), using the sensed data and/or power supply value in calculations and/or algorithms, or any combination of the above. After processing the, sensor data packet, the remote communication device 20 waits, as represented in block 208, either for a predetermined amount of time or for a request from another remote device (not shown) to request data from the second sensor member 24.

Referring to block 220, the remote communication device 20 broadcasts a message requesting a sensor data packet from the second sensor member 24 similar to the procedure described above related to step 120. The first sensor member 22 responds by providing a sensor data packet to the remote communication device 20 as described above and then returns to a power save state.

Referring to block 222, after sending the sensor data request message and waiting a predetermined length of time, if the remote communication device 20 has not received a sensor data packet from the second sensor member 24, the remote communication device will request information from the first sensor member 22, as is illustrated in block 202 and described above. Before the remote communication device 20 sends a sensor data request message to the first sensor member 22, the remote communication device 20 may attempt more than once to collect a sensor data packet from the second sensor member 24. If neither the first sensor member 22, nor the second sensor member 24 is responding, the remote communication device 20 will not continuously shift between requesting sensor data packets from the first and second sensor members, 22, 24, but will wait a predetermined time before attempting to establish communication with the first sensor member 22 after it has been determined that neither sensor is responding to data requests. If, however, the remote communication device 20 receives data from the second sensor member 24, the remote communication device processes the data as illustrated in block 224. The data received from the second sensor member 24 is processed in a similar manner to that described above with respect to the first sensor member 22. After processing data received from the second sensor member 24, the remote communication device 20 waits, as represented by a delay in block 226, either for a predetermined amount of time or for a request from another remote device (not shown) to move to step 202 and request data from the first sensor member 22.

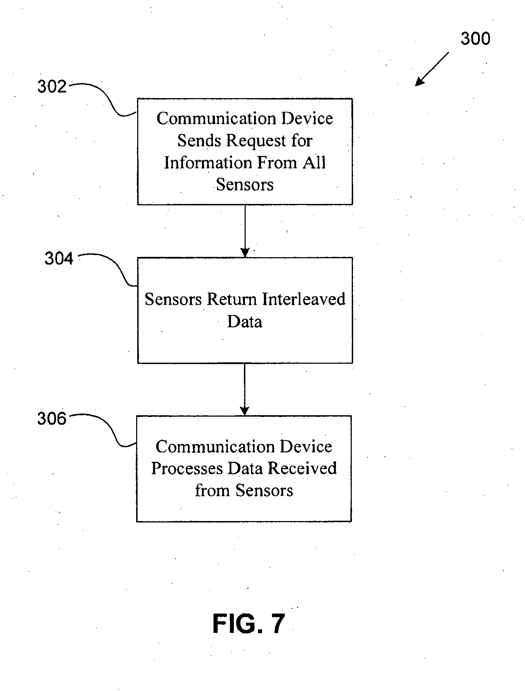

FIG. 7 is a functional flowchart 300 of a data collection method between the remote communication device 20 (FIG. 2) and the sensor members 12 (FIG. 2) according to another embodiment of the invention. In this embodiment, the remote communication device 20 requests a sensor data packet from each of the sensor members 12 simultaneously. A sensor data request can request data in different forms. For example, the sensor data request can specify that each of the sensors return a specified number of sensor data packets or the sensor members 12 return data values in a continuous stream of data for a given period of time. In response, the sensor members 12 transmit messages to the remote communications device 20. In some embodiments, sensor data values are transmitted sequentially in a specified order continuously from the time the remote communications device requests the sensor data until all of the data has been transmitted. In these and other embodiments, the data collection method may be performed in response to an external data request, or alternatively, it may be performed periodically.

Referring to block 302 of flowchart 300, prior to any communication between the remote communication device 20 and the sensor members 12, the sensor members are in a power save state. The remote communication device 20 requests a sensor data packet from each of the plurality of sensor members 12 by broadcasting a request for sensor data message including an address that corresponds to the global address 40 of each of the sensor members 12. In one embodiment, the message sent to request the sensor data from the sensor members 12 by the remote communication device 20 prescribes the order and for how long the sensor members 12 are to return information. For each sensor member 12, the remote communication device 20, in one embodiment, indicates how long after the request for sensor data a particular sensor should wait before making its first transmission, the delay between each subsequent transmission, and for how long the sensors are to return data after the request for sensor data.

As an example, a human body has two implanted sensor members 12 and it is desired that the two sensor members 12 transmit information for 10 seconds. Each sensor data packet transmission lasts 250 milliseconds. The first sensor member 22 will be instructed or will have stored within it to begin transmitting information at time t = 0 and begin a transmission every 500 milliseconds. The second sensor member 24 will be instructed to begin transmitting information at time t = 250 milliseconds and begin a transmission every 500 milliseconds thereafter. The request for data message may request continuous data for 10 seconds. Upon receipt of the request for data message, the first sensor member 22 begins transmission of its first sensor data packet at t = 0 seconds. The second sensor member 24 begins transmission at t = 250 milliseconds. The sensor members 12 then alternate sending packets every 250 milliseconds for 10 seconds so that the data returned from the sensor members 12 is interleaved. By staggering the transmissions, data collisions between two sensors attempting to transmit simultaneously are avoided. It should be understood that time t = 0 may be some period of time after the receipt of the data message to allow time for the sensor to collect data.

The specific time intervals in the example above are not intended to be limiting, as any time interval may be used, depending upon the amount of data to be collected, the number of sensors implanted in the body, and the length of time required to transmit each message from one of the sensor members 12 to the remote communication device 20.

Alternatively, the remote communication device 20 can provide each sensor member 12 with specific information regarding its order prior to the request for data message sent to each of the sensor members individually, by transmitting a series of messages each of which has an address that corresponds to one of the unique addresses 38 (FIG. 3) of the sensor members 12. The information provided by the remote communication device 20 to each sensor member 12 includes when to begin transmitting packets after receiving the broadcast message requesting sensor data, how much time the sensor member 12 should wait before transmitting that information and how often to transmit the packets. Alternatively still, each sensor members 12 includes specific information stored within the sensor member regarding when to begin transmitting messages after receiving the broadcast message requesting sensor data packets and how often to transmit the messages, therefore not requiring any communication from the remote communication device 20 to set the order of transmission. Once communication is initiated by the remote communication device 20, the sensor members 12 are in an identification state. After receiving the request for sensor data message, the sensor members 12 are in an active state.

Referring to block 304, the sensor members 12 transmit sensor data to the remote communication device 20 using the interleaving pattern of staggering data transmissions described above. The information is sent for the period of time defined in the request for data. Once the sensor data has been transmitted to the remote communication device 20, the sensor members 12 return to a power save state. Referring to block 306, the data sent by the sensor members 12 and received by the remote communication device 20 is processed by the remote communication device 20. Processing data can include storing the sensor packet data within the remote communication device 20, communicating the sensor data to another remote device (not shown in FIG. 2), using the sensor packet data in calculations and/or algorithms, or any combination of the above.

By measuring pressure pulses at each sensor member 12, for example, in a pulmonary artery, given that the distance between the sensor members 12 is known, it is possible to calculate mass blood flow through the artery using the data from the pulses over time. A particular method for calculating blood flow rate is described in U.S. Patent 6,277,078, issued August 21, 2001, and entitled "System and Method for Monitoring a Parameter Associated With the Performance of a Heart". In one embodiment, the data collection method illustrated in flowchart 300 and described above is initiated in response to an external request. Alternatively, the data collection method is performed periodically and is initiated after a specified period of time. While the remote communication device 20 may collect data periodically to measure mass blood flow, it need not use the data collection method described here exclusively. For example, the remote communication device 20 can use the method described in either the flowchart 100 or flowchart 200 primarily and intersperse the method described in flowchart 300 every 100 times data is collected or any other frequency.

In view of the foregoing, various embodiments are advantageous from several respects. For example, although optionally rechargeable, repeated recharging events and power drainage the power supplies can degrade and eventually be unable to maintain a charge. By having two or more sensors implanted in close proximity to one another, either of the sensors can be used to take readings, thereby reducing the overall usage of each sensor, and reducing the need for recharging events and, as a result, extending the life of sensors.

As another example, with the sensor assembly 10 according to various embodiments, it is possible to get sensors positioned more closely together where it would otherwise be difficult to implant two sensors close together without disturbing one or both of the sensors.

As another example, because the sensors of the assembly 10 are spaced apart at a known fixed distance according to some embodiments, it is possible to make calculations of mass blood flow by measuring the pulses as they pass each of the sensors as described above.

As a final example, because embodiments include multiple sensors (e.g., two), a redundant backup optionally exists for safety purposes.

Various modifications and additions can be made to the exemplary embodiments discussed without departing from the scope of the present invention. For example, while the embodiments described above refer to particular features, the scope of this invention also includes embodiments having different combinations of features and embodiments that do not include all of the described features. Accordingly, the scope of the present invention is intended to embrace all such alternatives, modifications, and variations as fall within the scope of the appended claims.

## Claims

1. A pressure sensing system, comprising:
a remote communication device (20);
a pressure sensing assembly (10) for implantation into a human body, the pressure sensing assembly (10) including:
a flexible structure (16) capable of expanding when implanted within the human body;
a first pressure sensor member (12, 22) coupled to the flexible structure, the first pressure sensor member including:
a self-contained power supply (30);
a sensing element (32); and
an integral communication device (36) capable of communicating with the remote communication device (20); and
a second pressure sensor member (12, 24) coupled to the flexible structure;
**characterized in that** the remote communication device (20) is operable to transmit a sensor data request message to the first and second pressure sensor members (22, 24), the sensor data request message being configured to prompt each pressure sensor member (12) to transition from a power save state to an initialization state for processing incoming communication data;
and **in that** each pressure sensor member (12) is operable to compare an address in the sensor data request message against a unique address and, upon receiving a matching address, transition from the initialization state to an active state in which said pressure sensor member is activated for collecting pressure data; and
and **in that** the remote communication device is further operable to collect and process a sensor data packet transmitted from one of the sensor members, the sensor data packet including a power supply value describing the charge level of a power supply of the one of the sensor members.

2. The pressure sensing system of claim 1, wherein the flexible structure (16) is a stent.

3. The pressure sensing system of claim 1, wherein the flexible structure (16) has a first end and a second end, and wherein the first pressure sensor member (12, 22) is coupled to the first end.

4. The pressure sensing system of claim 3, wherein the second pressure sensor member (12, 24) is coupled to the second end of the flexible structure (16).

5. The pressure sensing system of claim 1, wherein the remote communication device (20) is configured to be implanted within the human body.

6. The pressure sensing system of claim 5, wherein the remote communication device (20) is a pulse generator.

7. The pressure sensing system of claim 1, wherein the remote communication device (20) is an external device configured to be located outside of the human body.

8. The pressure sensing system of claim 1, wherein the integral communication device (36) of the first pressure sensor member (12, 22) is configured to wirelessly communicate with the remote communication device (20) via an acoustic communication link.

9. The pressure sensing system of claim 1, wherein the integral communication device (36) of the first pressure sensor member (12, 22) is configured to wirelessly communicate with the remote communication device via an inductive or radiofrequency communication link.

10. The pressure sensing system of claim 1, wherein the second pressure sensor member includes:
a self-contained power supply (30);
a sensing element (32); and
an integral communication device (36) capable of communicating with the remote communication device (20).

11. The pressure sensing system of claim 10, wherein the integral communication device (36) of the second pressure sensor member (12, 24) is configured to wirelessly communicate with the remote communication device (20) via an acoustic communication link.

12. The pressure sensing system of claim 10, wherein the integral communication device (36) of the second pressure sensor member (12, 24) is configured to wirelessly communicate with the remote communication device (20) via an inductive or radiofrequency communication link.

13. The pressure sensing system of claim 1, wherein the remote communication device (20) is capable of broadcasting messages having address data, and wherein the first pressure sensor member (12, 22) includes a memory unit with address data and is capable of recognizing messages broadcast from the remote communication device (20) having corresponding address data.

14. The pressure sensor system of claim 1, wherein the pressure sensing assembly is adapted to be implanted within a bodily vessel through a catheter.

15. The pressure sensor system of claim 1, wherein the remote communication device is operable to compare the power supply value of the one of the sensor members against a threshold value.

## Patentansprüche

1. Druckerfassungssystem, welches aufweist:
eine entfernte Kommunikationsvorrichtung (20);
eine Druckerfassungsanordnung (10) zur Implantierung in einen menschlichen Körper, welche Druckerfassungsanordnung (10) enthält:
eine flexible Struktur (16), die in der Lage ist, sich auszudehnen, wenn sie innerhalb des menschlichen Körpers implantiert ist;
ein erstes Drucksensorteil (12, 22), das mit der flexiblen Struktur gekoppelt ist, wobei das erste Drucksensorteil enthält:
eine selbständige Energieversorgung (30);
ein Erfassungselement (32); und
eine integrale Kommunikationsvorrichtung (36), die in der Lage ist, mit der entfernten Kommunikationsvorrichtung (20) zu kommunizieren; und
ein zweites Drucksensorteil (12, 24), das mit der flexiblen Struktur gekoppelt ist;
**dadurch gekennzeichnet, dass**
die entfernte Kommunikationsvorrichtung (20) betätigbar ist, eine Sensordaten-Anforderungsnachricht zu dem ersten und dem zweiten Drucksensorteil (22, 24) zu übertragen, wobei die Sensordaten-Anforderungsnachricht ausgebildet ist, jedes Drucksensorteil (12) zu veranlassen, aus einem Energiesparzustand in einen Initialisierungszustand für die Verarbeitung eintreffender Kommunikationsdaten überzugehen;
und dass jedes Drucksensorteil (12) betätigbar ist, eine Adresse in der Sensordaten-Anforderungsnachricht mit einer einmaligen Adresse zu vergleichen und bei Empfang einer übereinstimmenden Adresse aus dem Initialisierungszustand in einen aktiven Zustand, in welchem das Drucksensorteil für das Sammeln von Druckdaten aktiviert ist, überzugehen;
und dass die entfernte Kommunikationsvorrichtung weiterhin betätigbar ist zum Sammeln und Verarbeiten eines von einem der Sensorteile übertragenen Sensordatenpakets, wobei das Sensordatenpaket einen Energieversorgungswert enthält, der den Ladepegel einer Energieversorgung eines der Sensorteile beschreibt.

2. Druckerfassungssystem nach Anspruch 1, bei dem die flexible Struktur (16) ein Stent ist.

3. Druckerfassungssystem nach Anspruch 1, bei dem die flexible Struktur (16) ein erstes Ende und ein zweites Ende hat und bei dem das erste Drucksensorteil (12, 22) mit dem ersten Ende gekoppelt ist.

4. Druckerfassungssystem nach Anspruch 3, bei dem das zweite Drucksensorteil (12, 24) mit dem zweiten Ende der flexiblen Struktur (16) gekoppelt ist.

5. Druckerfassungssystem nach Anspruch 1, bei dem die entfernte Kommunikationsvorrichtung (20) ausgebildet ist, innerhalb des menschlichen Körpers implantiert zu sein.

6. Druckerfassungssystem nach Anspruch 5, bei dem die entfernte Kommunikationsvorrichtung (20) ein Impulsgenerator ist.

7. Druckerfassungssystem nach Anspruch 1, bei dem die entfernte Kommunikationsvorrichtung (20) eine externe Vorrichtung ist, die ausgebildet ist, außerhalb des menschlichen Körpers angeordnet zu sein.

8. Druckerfassungssystem nach Anspruch 1, bei dem die integrale Kommunikationsvorrichtung (36) des ersten Drucksensorteils (12, 22) ausgebildet ist, über eine akustische Kommunikationsverbindung drahtlos mit der entfernten Kommunikationsvorrichtung (20) zu kommunizieren.

9. Druckerfassungssystem nach Anspruch 1, bei dem die integrale Kommunikationsvorrichtung (36) des ersten Drucksensorteils (12, 22) ausgebildet ist, über eine induktive oder Hochfrequenz-Kommunikationsverbindung drahtlos mit der entfernten Kommunikationsvorrichtung zu kommunizieren.

10. Druckerfassungssystem nach Anspruch 1, bei dem das zweite Drucksensorteil enthält:
eine selbständige Energieversorgung (30);
ein Erfassungselement (32); und
eine integrale Kommunikationsvorrichtung (36), die in der Lage ist, mit der entfernten Kommunikationsvorrichtung (20) zu kommunizieren.

11. Druckerfassungssystem nach Anspruch 10, bei dem die integrale Kommunikationsvorrichtung (36) des zweiten Drucksensorteils (12, 24) ausgebildet ist, über eine akustische Kommunikationsverbindung drahtlos mit der entfernten Kommunikationsvorrichtung (20) zu kommunizieren.

12. Druckerfassungssystem nach Anspruch 10, bei dem die integrale Kommunikationsvorrichtung (36) des zweiten Drucksensorteils (12, 24) ausgebildet ist, über eine induktive oder Hochfrequenz-Kommunikationsverbindung drahtlos mit der entfernten Kommunikationsvorrichtung (20) zu kommunizieren.

13. Druckerfassungssystem nach Anspruch 1, bei dem die entfernte Kommunikationsvorrichtung (20) in der Lage ist, eine Rundsendung von Nachrichten mit Adressendaten durchzuführen, und bei dem das erste Drucksensorteil (12, 22) eine Speichereinheit mit Adressendaten enthält und in der Lage ist, von der entfernten Kommunikationsvorrichtung (20) rundgesendete Nachrichten mit entsprechenden Adressendaten zu erkennen.

14. Drucksensorsystem nach Anspruch 1, bei dem die Druckerfassungsanordnung ausgebildet ist, durch ein Katheter innerhalb eines Körpergefäßes implantiert zu werden.

15. Drucksensorsystem nach Anspruch 1, bei dem die entfernte Kommunikationsvorrichtung betätigbar ist, um den Energieversorgungswert des einen der Sensorteile mit einem Schwellenwert zu vergleichen.

## Revendications

1. Système de détection de pression, comprenant :
un dispositif de communication à distance (20) ;
un assemblage de détection de pression (10) destiné à être implanté dans un corps humain, l'assemblage de détection de pression (10) incluant :
une structure flexible (16) pouvant s'étendre lorsqu'elle est implantée dans le corps humain ;
un premier élément de capteur de pression (12, 22) couplé à la structure flexible, le premier élément de capteur de pression incluant :
une alimentation autonome (30) ;
un élément de détection (32) ; et
un dispositif de communication intégré (36) pouvant communiquer avec le dispositif de communication à distance (20) ; et
un second élément de capteur de pression (12, 24) couplé à la structure flexible, **caractérisé en ce que** :
le dispositif de communication à distance (20) peut fonctionner pour transmettre un message de requête de données de capteur sur les premier et second éléments de capteur de pression (22, 24), le message de requête de données de capteur étant configuré pour demander à chaque élément de capteur de pression (12) de réaliser une transition depuis un état d'économie d'énergie dans un état d'initialisation pour traiter des données de communication arrivantes ; et **en ce que**
chaque élément de capteur de pression (12) peut fonctionner pour comparer une adresse dans le message de requête de données de capteur avec une adresse unique et, suite à la réception d'une adresse en correspondance, pour réaliser une transition depuis l'état d'initialisation dans un état actif dans lequel ledit élément de capteur de pression est activé pour collecter des données de pression ; et **en ce que**
le dispositif de communication à distance peut en outre fonctionner pour collecter et traiter un paquet de données de capteur transmis depuis l'un des éléments de capteur, le paquet de données de capteur incluant une valeur d'alimentation décrivant le niveau de charge d'une alimentation de l'un des éléments de capteur.

2. Système de détection de pression selon la revendication 1, dans lequel la structure flexible (16) est un stent.

3. Système de détection de pression selon la revendication 1, dans lequel la structure flexible (16) comporte une première extrémité et une seconde extrémité et dans lequel le premier élément de capteur de pression (12, 22) est couplé à la première extrémité.

4. Système de détection de pression selon la revendication 3, dans lequel le second élément de capteur de pression (12, 24) est couplé à la seconde extrémité de la structure flexible (16).

5. Système de détection de pression selon la revendication 1, dans lequel le dispositif de communication à distance (20) est configuré de manière à pouvoir être implanté dans le corps humain.

6. Système de détection de pression selon la revendication 5, dans lequel le dispositif de communication à distance (20) est un générateur d'impulsions.

7. Système de détection de pression selon la revendication 1, dans lequel le dispositif de communication à distance (20) est un dispositif externe configuré de manière à pouvoir être placé à l'extérieur du corps humain.

8. Système de détection de pression selon la revendication 1, dans lequel le dispositif de communication intégré (36) du premier élément de capteur de pression (12, 22) est configuré de manière à pouvoir communiquer sans fil avec le dispositif de communication à distance (20) via une liaison de communication acoustique.

9. Système de détection de pression selon la revendication 1, dans lequel le dispositif de communication intégré(36) du premier élément de capteur de pression (12, 22) est configuré de manière à pouvoir communiquer sans fil avec le dispositif de communication à distance via une liaison de communication par induction ou radiofréquences.

10. Système de détection de pression selon la revendication 1, dans lequel le second élément de capteur de pression inclut :
une alimentation autonome (30) ;
un élément de détection (32) ; et
un dispositif de communication intégré (36) permettant de communiquer avec le dispositif de communication à distance (20).

11. Système de détection de pression selon la revendication 10, dans lequel le dispositif de communication intégré (36) du second élément de capteur de pression (12, 24) est configuré de manière à pouvoir communiquer sans fil avec le dispositif de communication à distance (20) via une liaison de communication acoustique.

12. Système de détection de pression selon la revendication 10, dans lequel le dispositif de communication intégré (36) du second élément de capteur de pression (12, 24) est configuré de manière à pouvoir communiquer sans fil avec le dispositif de communication à distance (20) via une liaison de communication par induction ou radiofréquences.

13. Système de détection de pression selon la revendication 1, dans lequel le dispositif de communication à distance (20) permet de diffuser des messages comportant des données d'adresse et dans lequel le premier élément de capteur de pression (12, 22) inclut une unité de mémoire munie de données d'adresse et permet de reconnaître des messages diffusés depuis le dispositif de communication à distance (20) comportant des données d'adresse en correspondance.

14. Système de capteur de pression selon la revendication 1, dans lequel l'assemblage de détection de pression est adapté pour être implanté dans un vaisseau du corps au travers d'un cathéter.

15. Système de capteur de pression selon la revendication 1, dans lequel le dispositif de communication à distance peut fonctionner pour comparer la valeur d'alimentation de l'un des éléments de capteur avec une valeur de seuil.
